# EUROPÄISCHE PATENTSCHRIFT

(11) **EP 0 602 482 B1**
(45) Veröffentlichungstag und Bekanntmachung des Hinweises auf die Patenterteilung: **26.07.1995**
(21) Anmeldenummer: 93119570.5
(22) Anmeldetag: 04.12.1993
(51) Int. Cl.: C07C 21/18, C07C 17/26

(54) **Hochreines Perfluor-(4-methyl-2-penten), seine Herstellung und Verwendung**
Perfluoro-(4-methyl-2-pentene) of high purity, its preparation and use
Perfluoro-(4-méthyl-2-pentène) d'haute pureté, sa préparation et son application

(30) Priorität: 12.12.1992 DE 4241969
(43) Veröffentlichungstag der Anmeldung: 22.06.1994
(73) Patentinhaber: HOECHST AKTIENGESELLSCHAFT, 65926 Frankfurt am Main (DE)
(72) Erfinder: Von Werner, Konrad, Dr., D-84518 Garching (DE)

(56) Entgegenhaltungen:
- EP-A- 0 367 256
- US-A- 2 918 501
- US-A- 3 917 724
- US-A- 4 042 638
- CHEMISCHE BERICHTE, Bd.106, Nr.9, 1973 Seiten 2950 - 2959 SIGMAR P. V. HALASZ ET AL. 'Darstellung und Fluorierung von Oligomeren des Hexafluorpropens'
- CHEMICAL ABSTRACTS, vol. 83, no. 19, 10. November 1975, Columbus, Ohio, US; abstract no. 163550g, ISHIKAWA NOBUO ET AL. 'Preparation of trans-perfluoro-4-methyl-2-pentene and perfluoro-2-methyl-2-pentene' Seite 495 ;Spalte 2 ;

## Beschreibung

Die Erfindung betrifft ein Perfluor-(4-methyl-2-penten), im folgenden (I), mit einem Gehalt an Perfluor-(2-methyl-2-penten), im folgenden (II), unter 1000 ppm, vorzugsweise unter 100 ppm und insbesondere unter 10 ppm. Die Erfindung betrifft weiterhin die Herstellung eines derart hochreinen (I) durch Abtrennung von (II) aus entsprechenden Ausgangsmaterialien. Die Erfindung bezieht sich weiterhin auf die Verwendung von hochreinem (I) als Substitutionsprodukt für Fluorchlorkohlenwasserstoffe.

(I), insbesondere das trans-Isomere, ist das Hauptprodukt bei der Dimerisierung von Hexafluorpropen. Bei allen bekannten Dimerisierungsverfahren für Hexafluorpropen wird jedoch ein mehr oder weniger hoher Anteil an (II) gebildet, das inhalationstoxisch ist.

Es wurde bereits ein Verfahren zur Herstellung von Dimeren des Hexafluorpropens mit einem hohen Anteil an (I) und einem niedrigen Gehalt an (II) vorgeschlagen, das dadurch gekennzeichnet ist, daß die Dimerisierung des Hexafluorpropens in einem aprotischen Lösemittel in Gegenwart eines Adduktes aus einem Amin, welches keine NH-Gruppen enthält, und einem Metallfluorid erfolgt (europäischen Patentanmeldung EP-A-588 106). Nach diesem Verfahren sind Dimerisierungsprodukte erhältlich, deren Gehalt an (II) auf bis zu 0,2 % reduziert wurde.

Es wurde nun gefunden, daß der Gehalt an (II) in Gemischen mit überwiegend (I) auf einen Gehalt an unter 1000 ppm (II) verringert werden kann, wenn die Mischungen mit niederen Alkanolen und katalytischen Mengen einer Base behandelt werden. Hierbei wird (II) in hochsiedende Verbindungen übergeführt, die leicht destillativ von (I) getrennt werden können. Bevorzugte Ausführungsformen dieser Erfindung werden im folgenden näher erläutert.

Als Basen sind grundsätzlich Alkalimetallhydroxide wie NaOH oder KOH geeignet, wenn man bei schonenden Bedingungen, also vor allem bei tiefen Temperaturen, arbeitet, da sonst die Selektivität der Reaktion verringert wird. Geeignet sind Alkali- und Ammoniumcarbonate und -hydrogencarbonate wie Natriumcarbonat, Kalium- oder Tetramethylammoniumhydrogencarbonat. Weiterhin können Metallfluoride, insbesondere Alkalimetallfluoride wie KF verwendet werden, wobei aber zu beachten ist, daß dann Fluoridionen in das Waschwasser gelangen. Besonders bevorzugt sind tertiäre Amine, die bereits in sehr kleinen Mengen wirken und eine hohe Selektivität gewährleisten, wie beispielsweise niedere Trialkylamine oder peralkylierte, chelatbildende Diamine oder Triamine, die in den Alkyl- oder Alkylengruppen auch Ethersauerstoff enthalten können. Derartige Amine wurden für das Verfahren der europäischen Patentanmeldung EP-A-588 106 vorgeschlagen. Bevorzugt werden 0,05 bis 0,1 mol Amin pro mol (II).

Bevorzugte Ausgangsmaterialien sind Produkte des in der europäischen Patentanmeldung 93113408.4 vorgeschlagenen Verfahrens. Diese Produkte enthalten einerseits nur geringe Mengen an (II) und andererseits - als Rohprodukte - bereits geeignete Amine und gegebenenfalls Fluoride als auch für das erfindungsgemäße Verfahren geeignete Katalysatoren.

Es können aber auch andere Ausgangsmaterialien mit einem überwiegenden Gehalt an (I) neben (II) eingesetzt werden, wie sie beispielsweise nach der US-A 2 918 501 durch Oligomerisierung von Hexafluorpropen mit Haliden und Lösemitteln aus der Klasse der alkylsubstituierten Amide, Phenylamine und Sulfoxide erhältlich sind. Bei weiteren bekannten Verfahren zur Di- und Trimerisierung von Hexafluorpropen werden als Katalysatoren tris-[2-(2H-Hexafluorpropoxy)-ethyl]-amin in Acetonitril [Halasz et al., Chem. Ber. 106 (1973) 2950 bis 2955] oder Komplexsalze eines Alkalimetallhalogenids mit einem Kronenether (US-A 4 042 638) eingesetzt.

Als niedere Alkanole sind alle Verbindungen geeignet, die mit (II) hochsiedende Verbindungen bilden können. Zweckmäßig wählt man gut abtrennbare Alkanole, also beispielsweise leicht wasserlösliche Verbindungen. Geeignet sind Alkanole mit 1 bis 6 Kohlenstoffatomen, wobei der Alkylrest linear, verzweigt oder cyclisch sein kann. Bevorzugt sind Alkanole mit 1 bis 3 Kohlenstoffatomen, vor allem Methanol, da dieses besonders rasch und selektiv reagiert.

Bevorzugt wird das Alkanol im stöchiometrischen Überschuß, bezogen auf den Gehalt an (II), eingesetzt. Vorteilhaft ist ein mehr oder weniger großer Überschuß, da die Hauptmenge des Alkanols nach der Reaktion sich als Oberphase abscheidet und deshalb leicht abgetrennt und in das Verfahren zurückgeführt werden kann. In der Praxis wählt man etwa 5 bis 15 Vol.-% an Alkanol, bezogen auf das Volumen des zu reinigenden (I) beziehungsweise des Oligomerisierungsproduktes von Hexafluorpropen.

Die Behandlung des Ausgangsmaterials mit dem Alkanol erfolgt in flüssiger Phase, zweckmäßig bei Temperaturen von etwa -20 °C bis etwa 40 °C, vorteilhaft bei 0 °C bis etwa 35 °C, bevorzugt bei 15 bis 30 °C, insbesondere bei 20 bis 25 °C.

Da das Ausgangsprodukt mit dem niederen Alkanol ein zweiphasiges Gemisch bildet, muß für einen guten Kontakt zwischen dem Alkanol und (II) gesorgt werden. Intensives Rühren oder Schütteln in üblichen Apparaten verkürzt die Reaktionszeit.

Der Grad der Umsetzung kann mit üblichen analytischen Mitteln, beispielsweise gaschromatographisch, verfolgt werden. Die Behandlungszeit richtet sich selbstverständlich nach der gewünschten Verringerung des Gehaltes an (II), nach der Reaktivität des Alkanols und der Wirksamkeit des Katalysators. Üblicherweise genügen Zeiten von bis zu 6 Stunden, meist bis zu 3 Stunden, um den Gehalt an (II) auf unter 10 ppm zu senken.

Am Ende der Behandlung läßt man die Reaktionsmischung sich in zwei Phasen auftrennen und entfernt die Alkoholphase. Diese kann ohne weitere Reinigung für einen neuen Ansatz verwendet werden.

Die (I) enthaltende Phase wird destillativ von den aus (II) gebildeten hochsiedenden Produkten getrennt. Man kann so ein (I) erhalten, dessen Gehalt an (II) unter der analytischen Nachweisgrenze liegt.

Das erfindungsgemäß erhältliche hochreine (I) ist üblicherweise ein cis-trans-Gemisch mit etwa 95 % trans-Anteil. Es kann noch Anteile an Hexafluorpropen-Trimeren enthalten, die üblicherweise beim Einsatz als Substitutionsprodukte für Fluorchlorkohlenwasserstoffe nicht stören.

Das erfindungsgemäß erhältliche (I) eignet sich insbesondere als Wärmeträger, Kühl- und Isoliermedium, vor allem für die zweiphasige Siedekühlung von elektrischen und elektronischen Bauelementen, beispielsweise als Kühlmedium für die zweiphasige Siedekühlung von Gasen oder Flüssigkeiten ohne Hochspannungsisolation, vorzugsweise in einem Wärmerohr, oder als Wärmeträgermedium, wobei die Wärmeenergie durch einphasige Konvektion von dem zu kühlenden gasförmigen, flüssigen oder festen Stoff zu einem anderen zu erwärmenden gasförmigen, flüssigen oder festen Stoff geführt wird (europäische Patentanmeldung 93115932.1 vom 02.10.1993).

In den folgenden Beispielen wird die Erfindung näher erläutert.

### Beispiel 1

### Herstellung von Hexafluorpropen(HFP)-Dimeren:

Diese Versuche erfolgten in einem 16-1-Kessel aus elektropoliertem Edelstahl, der mit einem regelbaren Rührer, einer geeichten Zuführung für gasförmiges Hexafluorpropen sowie Dosierungen für Flüssigkeiten und Feststoffe ausgerüstet ist. Heizung und Kühlung des Kessels kann über einen Kreislauf mit einem externen Platten-Wärmetauscher auf ± 1 °C genau geregelt werden.

Zunächst wird im Labor eine Katalysatorlösung aus folgenden Komponenten hergestellt:
- 1,5 l Acetonitril (entwässert durch Destillation über etwas Calciumhydrid und Aufbewahren über 3-Å-Molekularsieb),
- 69,7 g (0,6 mol) N,N,N',N'-Tetramethylethylendiamin (getrocknet über 4-Å-Molekularsieb),
- 34,9 g (0,6 mol) Kaliumfluorid-Pulver (getrocknet durch Rühren des Pulvers bei 180 °C/0,5 mbar).

Die Komponenten werden unter trockenem Stickstoff in dieser Reihenfolge bei laufendem Rührer zusammengegeben und bei 30 bis 40 °C 1 Stunde intensiv gerührt. Man erhält eine gelbliche Lösung, die kaum noch festes Kaliumfluorid enthält.

Diese Lösung wird in den sorgfältig mit N₂ gespülten 16-1-Kessel eingefüllt. Man heizt den Kessel bei geschlossenem Kopfventil auf 40 °C auf und dosiert dann innerhalb von 6 Stunden bei laufendem Rührer 16 kg gasförmiges HFP zu. Die Reaktion ist leicht exotherm. Die Innentemperatur wird durch Kühlung bei 40 bis 50 °C gehalten. Danach läßt man noch 2 Stunden bei 40 °C nachreagieren und kühlt dann die Mischung bei abgestelltem Rührer auf 15 °C ab.

Über das Bodenventil des Kessels werden langsam 14 kg Rohprodukt in einen Behälter abgelassen, der zum Druckausgleich mit der HFP-Zuleitung zum Kessel verbunden ist (Pendelleitung). 2 kg des Produktes verbleiben mit der leichteren Katalysatorphase im Kessel.

Das Rohprodukt wird durch Gaschromatographie analysiert. Zur Analyse wird eine gepackte Säule (5 % GEXE 60 auf ®PORASIL C) mit einem Wärmeleitfähigkeits-Detektor verwendet (Trägergas: Helium). Die Zuordnung der Komponenten wurde vorher durch Bestimmung der Retentionszeiten und Flächenfaktoren mit weitgehend reinen Referenzverbindungen ermittelt. Man erhält folgende Verteilung der fluorhaltigen Produkte:

| | |
|---|---|
| CF₃-CF=CF₂ | 0,2 % |
| (CF₃)₂CFH | 0,1 % |
| trans-(CF₃)₂CF-CF=CF-CF₃ (I) | 90,1 % |
| cis-(CF₃)₂CF-CF=CF-CF₃ (I) | 5,0 % |
| (CF₃)₂C=CF-CF₂CF₃ (II) | 0,4 % |
| Σ HFP-Trimere | 4,2 % |

Durch ¹⁹F-NMR-Analyse des Rohprodukts bei 282,4 MHz (Lösung in CDCl₃/R113, Standard: CF₃CO₂H) wurde bestätigt, daß das durch GC bestimmte Verhältnis von trans- und cis-(I)-Dimeren dem Molverhältnis dieser beiden Verbindungen entspricht. Dies wurde durch die Auswertung der Integrale der an die Doppelbindung gebundenen Fluoratome ermittelt. Nachfolgend sind die ¹⁹F-NMR-Kenndaten der beiden Isomeren aufgeführt (Standard: Trifluoressigsäure). 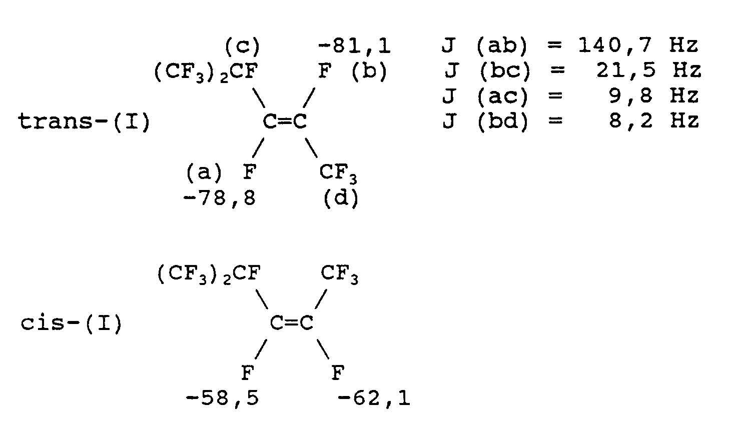
Mit der im Kessel verbliebenen Katalysatorlösung wurden noch zwei weitere Chargen durchgeführt, wobei jeweils 14 kg HFP umgesetzt wurden. Nach der insgesamt dritten Charge wurde die Katalysatorlösung vom Produkt abgetrennt.

Die GC-Analyse ergab folgende Produktverteilungen:

| | Charge 2 | Charge 3 |
|---|---|---|
| CF₃CF=CF₂ | 0,5 % | 1,0 % |
| (CF₃)₂CFH | 0,1 % | 0,2 % |
| trans-(CF₃)₂CF-CF=CF-CF₃ (I) | 90,1 % | 90,3 % |
| cis-(CF₃)₂CF-CF=CF-CF₃ (I) | 5,1 % | 5,0 % |
| (CF₃)₂C=CF-CF₂CF₃ (II) | 0,4 % | 0,2 % |
| (CF₃)₂CH-CF₂CF₂CF₃ | 0,1 % | 0,1 % |
| Σ HFP-Trimere | 3,7 % | 3,2 % |

Das hochtoxische Perfluor-2-methyl-1-penten konnte in keiner Charge nachgewiesen werden.

Insgesamt wurden durch Vereinigung der Rohprodukte 43,5 kg erhalten. Dieses Material enthält noch Spuren von Verunreinigungen, die aus der Katalysatorlösung stammen (unter anderem circa 0,03 % Tetramethylethylendiamin).

### Erfindungsgemäße Reinigung:

Das vorstehend beschriebene Rohprodukt wurde mit 3 l Methanol während 2 Stunden bei circa 20 °C intensiv gerührt. Anschließend wurden die Phasen getrennt. Die Produktphase wurde durch Gegenstromwäsche in einer mit Raschigringen gefüllten vertikalen Blasensäule gereinigt. Die Säule war mit 10 l Wasser gefüllt. Das Fluorprodukt wurde am Kopf zudosiert und über die Säule verrieselt. Gleichzeitig wurden im Gegenstrom 20 l Wasser am unteren Ende eingebracht. Das gereinigte Produkt sammelt sich am unteren Ende der Säule in einem Auffanggefäß. Nach Abtrennen einer überstehenden Wasserschicht enthielt das Produkt (43 kg) weniger als 0,005 % Wasser.

Zur abschließenden Reinigung wurde das gewaschene Produkt bei Normaldruck über eine Füllkörperkolonne destilliert, deren Kondenser und Auffangbehälter mit Hilfe eines Kühlaggregates auf 0 °C gekühlt wurde. Als Hauptfraktion wurden 40 kg mit einem Siedebereich von 45,5 bis 49,0 °C erhalten. Zusammensetzung nach GC:

| | |
|---|---|
| trans-(I) | 94,7 % |
| cis-(I) | 5,3 % |

Der Gehalt an (II) liegt unter 10 ppm. Dies ist die Nachweisgrenze für (II) bei optimierter GC-Analytik. Die Masseausbeute beträgt 90 %, bezogen auf eingesetztes Hexafluorpropen.

### Beispiel 2

Es wurden 300 ml (482 g) eines Gemisches mit folgender Zusammensetzung verwendet:

| | |
|---|---|
| trans-(I) | 89,8 % |
| cis-(I) | 4,9 % |
| (II) | 5,2 % |
| HFP-Trimere | 0,1 % |

Diese Mischung wurde mit 30 ml absolutem Ethanol und 1,69 g Triethylamin [entsprechend 10 Mol-% bezüglich (II)] 4 Stunden bei circa 23 °C intensiv gerührt. Danach wurde die Mischung dreimal mit je 0,5 l Eiswasser geschüttelt. Die organische Phase wurde mit wenig Natriumsulfat getrocknet und dann bei Normaldruck über eine Kolonne destilliert, deren Auffangteil auf 0 °C gekühlt wurde. Als Hauptlauf wurden 426 g mit Siedebereich 46 bis 49 °C erhalten. Durch GC und ¹⁹F-NMR wurde folgende Zusammensetzung ermittelt:

| | |
|---|---|
| trans-(I) | 94,8 % |
| cis-(I) | 5,2 % |
| (II) | 320 ppm |

Der Destillationsrückstand wurde über eine Spaltrohrkolonne fraktioniert. Dabei wurden zunächst 6,2 g einer Fraktion mit Kp. 59 bis 61 °C erhalten, die als (CF₃)₂CH-CF₂CF₂CF₃ identifiziert wurde. Die bei 107 bis 110 °C übergehende Hauptfraktion (24,3 g) enthielt aufgrund ¹⁹F-NMR und ¹H-NMR die beiden folgenden Ether im Molverhältnis 2,1 : 1.

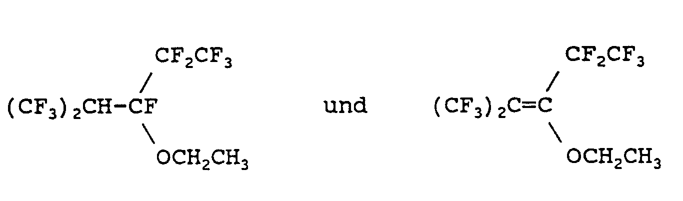

### Beispiel 3

Die Reaktion und Aufarbeitung wurde wie in Beispiel 2 durchgeführt, jedoch wurde Methanol anstelle von Ethanol verwendet. Das GC einer nach 2 Stunden entnommenen Probe zeigte, daß bereits zu diesem Zeitpunkt der Gehalt an (II) kleiner als 0,1 % ist. Nach 4 Stunden Reaktionszeit wurden nach Aufarbeitung erhalten:
439 g Hauptfraktion (Kp. 46 bis 48,5 °C), bestehend aus:

| | |
|---|---|
| trans-(I) | 94,9 % |
| cis-(I) | 5,1 % |
| (II) | 118 ppm |

2,8 g (CF₃)₂CH-CF₂CF₂CF₃ (Kp. 59 bis 62 °C)
13,5 g (Kp. 95 bis 98 °C) Gemisch aus 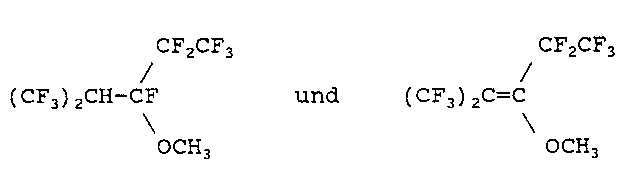
Die beiden Ether liegen im Molverhältnis 3,7 : 1 vor.

## Patentansprüche

1. Perfluor-(4-methyl-2-penten) mit einem Gehalt an Perfluor-(2-methyl-2-penten) unter 1000 ppm.

2. Perfluor-(4-methyl-2-penten) mit einem Gehalt an Perfluor-(2-methyl-2-penten) unter 100 ppm.

3. Perfluor-(4-methyl-2-penten) mit einem Gehalt an Perfluor-(2-methyl-2-penten) unter 10 ppm.

4. Verfahren zur Herstellung eines Perfluor-(4-methyl-2-pentens) mit einem Gehalt an Perfluor-(2-methyl-2-penten) unter 1000 ppm, dadurch gekennzeichnet, daß man ein Ausgangsmaterial aus überwiegend Perfluor-(4-methyl-2-penten) mit einem höheren Gehalt an Perfluor-(2-methyl-2-penten) mit einem niederen Alkanol und katalytischen Mengen einer Base behandelt und die aus dem Perfluor-(2-methyl-2-penten) gebildeten hochsiedenden Verbindungen abtrennt.

5. Verfahren nach Anspruch 4, dadurch gekennzeichnet, daß man das niedere Alkanol im stöchiometrischen Überschuß, bezogen auf den Gehalt an Perfluor-(2-methyl-2-penten), einsetzt.

6. Verfahren nach Anspruch 4 oder 5, dadurch gekennzeichnet, daß man ein Alkanol mit 1 bis 3 Kohlenstoffatomen einsetzt.

7. Verfahren nach einem oder mehreren der Ansprüche 4 bis 6, dadurch gekennzeichnet, daß die Behandlung mit dem Alkanol bei einer Temperatur von 0 bis 35 °C erfolgt.

8. Verfahren nach einem oder mehreren der Ansprüche 4 bis 7, dadurch gekennzeichnet, daß als Base ein tertiäres Amin eingesetzt wird.

9. Verfahren nach einem oder mehreren der Ansprüche 4 bis 7, dadurch gekennzeichnet, daß die Behandlung in einem Zeitraum von bis zu 3 Stunden erfolgt.

10. Verwendung der Verbindungen nach den Ansprüchen 1 bis 3 beziehungsweise der nach den Ansprüchen 4 bis 9 erhaltenen Produkte als Substitutionsprodukte für Fluorchlorkohlenwasserstoffe.

## Claims

1. Perfluoro-(4-methyl-2-pentene) having a content of perfluoro-(2-methyl-2-pentene) of below 1000 ppm.

2. Perfluoro-(4-methyl-2-pentene) having a content of perfluoro-(2-methyl-2-pentene) of below 100 ppm.

3. Perfluoro-(4-methyl-2-pentene) having a content of perfluoro-(2-methyl-2-pentene) of below 10 ppm.

4. A process for preparing a perfluoro-(4-methyl-2-pentene) having a content of perfluoro-(2-methyl-2-pentene)of below 1000 ppm, which comprises treating a starting material of mainly perfluoro-(4-methyl-2-pentene) having a higher content of perfluoro-(2-methyl-2-pentene) with a lower alkanol and catalytic amounts of a base and removing the high boiling-point compounds formed by reaction with the perfluoro-(2-methyl-2-pentene).

5. The process as claimed in claim 4, wherein the lower alkanol is used in a stoichiometric excess, based on the content of perfluoro-(2-methyl-2-pentene).

6. The process as claimed in claim 4 or 5, wherein an alkanol having from 1 to 3 carbon atoms is used.

7. The process as claimed in one or more of claims 4 to 6, wherein the treatment with the alkanol is carried out at a temperature of from 0 to 35°C.

8. The process as claimed in one or more of claims 4 to 7, wherein the base used is a tertiary amine.

9. The process as claimed in one or more of claims 4 to 7, wherein the treatment is carried out over a time period of up to 3 hours.

10. A method of using the compounds as claimed in any of claims 1 to 3 or the products obtained as claimed in any of claims 4 to 9 as substitutes for chlorofluorocarbons.

## Revendications

1. Perfluoro-(4-méthyl-2-pentène) ayant une teneur en perfluoro-(2-méthyl-2-pentène) inférieure à 1000 ppm.

2. Perfluoro-(4-méthyl-2-pentène) ayant une teneur en perfluoro-(2-méthyl-2-pentène) inférieure à 100 ppm.

3. Perfluoro-(4-méthyl-2-pentène) ayant une teneur en perfluoro-(2-méthyl-2-pentène) inférieure à 10 ppm.

4. Procédé de préparation d'un perfluoro-(4-méthyl-2-pentène) ayant une teneur en perfluoro-(2-méthyl-2-pentène) inférieure à 1000 ppm, caractérisé en ce que l'on traite un produit de départ constitué essentiellement de perfluoro-(4-méthyl-2-pentène) ayant une teneur élevée en perfluoro-(2-méthyl-2-pentène) avec un alcanol inférieur et des quantités catalytiques d'une base, et on sépare les composés de haut point d'ébullition formés à partir du perfluoro-(2-méthyl-2-pentène).

5. Procédé selon la revendication 4, caractérisé en ce que l'on utilise l'alcanol inférieur en un excès stoechiométrique par rapport à la teneur en perfluoro-(2-méthyl-2-pentène).

6. Procédé selon la revendication 4 ou 5, caractérisé en ce que l'on utilise un alcanol de 1 à 3 atomes de carbone.

7. Procédé selon l'une ou plusieurs des revendications 4 à 6, caractérisé en ce que l'on effectue le traitement avec l'alcanol à une température de 0 à 35°C.

8. Procédé selon l'une ou plusieurs des revendications 4 à 7, caractérisé en ce que l'on utilise une amine tertiaire comme base.

9. Procédé selon l'une ou plusieurs des revendications 4 à 7, caractérisé en ce que le traitement s'effectue en un laps de temps allant jusqu'à 3 heures.

10. Utilisation des composés selon les revendications 1 à 3 ou des produits obtenus selon les revendications 4 à 9 comme produits de substitution pour des hydrocarbures chlorofluorés.
